# FASCICULE DE BREVET EUROPEEN

(11) **EP 0 705 645 B1**
(45) Date de publication et mention de la délivrance du brevet: **22.03.2000**
(21) Numéro de dépôt: 95402194.5
(22) Date de dépôt: 29.09.1995
(51) Int. Cl.: B05B 11/00, A61F 9/00

(54) **Pompe à fluide sans volume mort**
Pumpe ohne Restvolumen zur Abgabe von Medien
Pump without dead volume for delivering a fluid

(30) Priorité: 03.10.1994 FR 9411785
(43) Date de publication de la demande: 10.04.1996
(73) Titulaire: Py, Daniel, Larchmont, New York 10538 (US)
(72) Inventeur: Py, Daniel, Larchmont, New York 10538 (US)
(74) Mandataire: Rinuy, Santarelli

(56) Documents cités:
- EP-A- 0 201 809
- EP-A- 0 492 354
- WO-A-93/10852
- US-A- 5 320 845

## Description

La présente invention concerne une pompe, notamment une pompe-flacon en trois pièces sans volume mort pour la délivrance d'un fluide, particulièrement sans conservateur et rempli stérilement.

US-A-5 320 845 décrit une pompe pour la délivrance d'un fluide contenu dans un flacon élastique comprenant :
- un corps de pompe ayant une extrémité antérieure ou buse du côté de la sortie du fluide, ladite extrémité antérieure comportant un orifice de sortie normalement obturé par une membrane élastique, et se poursuivant vers l'arrière par un conduit de pompe muni d'un orifice d'admission de fluide,
- un piston mobile installé dans le corps de pompe, le déplacement relatif de l'extrémité du piston par rapport au corps de pompe entre l'orifice d'admission et une position en butée située vers l'orifice de sortie déterminant ainsi la quantité de fluide expulsée lors du déplacement, l'extrémité du piston étant ajustée hermétiquement à frottement doux au conduit, l'orifice d'admission étant de taille suffisante pour que seule la quantité de fluide prédéterminée puisse se trouver capturée dans l'extrémité du conduit de pompe en vue de son expulsion par l'orifice de sortie.

Dans certaines industries, notamment dans l'industrie pharmaceutique, on recherche toujours des pompes permettant de délivrer un fluide, de préférence dénuées de volume mort de manière par exemple à éviter la contamination d'un fluide stérile, ou la dégradation d'un fluide fragile, au contact de l'oxygène de l'air, en produits toxiques ou inactifs.

Rappelons à cet effet que le volume mort est défini comme étant le volume compris entre le dispositif de fermeture d'une pompe, et l'air libre.

De plus il serait intéressant de disposer d'une pompe délivrant de manière régulière une quantité de fluide précise et, de préférence, quelque soit le volume considéré aussi bien des petits volumes de l'ordre de 10 µl que des volumes beaucoup plus importants.

En outre, de préférence la quantité de fluide délivrée ne devrait dépendre ni de la pesanteur, ni de la rapidité d'activation de la pompe.

Il serait également souhaitable de disposer d'une pompe dans laquelle l'absence d'air ou de conservateur dans le flacon dont est muni une telle pompe conserverait une formulation stable et de plus empêcherait la pollution de cette formulation, par exemple d'un milieu liquide sans conservateur.

De surcroît, un flacon muni d'une telle pompe devrait pouvoir bénéficier d'une exposition à l'air quasiment nulle pendant le remplissage du flacon, assurant ainsi la stérilité du contenu.

La réalisation de tels dispositifs est d'autant plus délicate que le volume à délivrer est petit, par exemple de l'ordre de 10 µl.

C'est pourquoi la présente invention a pour objet une pompe pour la délivrance d'un fluide contenu dans un flacon élastique comprenant :
- un corps de pompe ayant une extrémité antérieure ou buse du côté de la sortie du fluide, ladite extrémité antérieure comportant un orifice de sortie normalement obturé par une membrane élastique, et se poursuivant vers l'arrière par un conduit de pompe muni d'un orifice d'admission de fluide,
- un piston mobile installé dans le corps de pompe, le déplacement relatif de l'extrémité du piston par rapport au corps de pompe entre l'orifice d'admission et une position en butée située vers l'orifice de sortie déterminant ainsi la quantité de fluide expulsée lors du déplacement, l'extrémité du piston étant ajustée hermétiquement à frottement doux au conduit, l'orifice d'admission étant de taille suffisante pour que seule la quantité de fluide prédéterminée puisse se trouver capturée dans l'extrémité du conduit de pompe en vue de son expulsion par l'orifice de sortie, caractérisé en ce que le corps de pompe et le piston sont totalement enveloppés par le flacon élastique, a l'exception de l'extrémité antérieure du corps de pompe et en ce que l'activation de la pompe est effectuée au travers dudit flacon élastique.

Le fluide peut être par exemple une pâte, mais de préférence un gel et tout particulièrement un liquide.

L'extrémité antérieure du corps de pompe, buse ou "nez", comporte un orifice de sortie constitué de préférence d'un canal, par exemple cylindrique, débouchant dans un conduit de pompe, ce dernier étant par exemple classiquement en forme de tube cylindrique, orifice ou canal de sortie et conduit de pompe ayant de préférence la même direction générale. L'orifice de sortie est de préférence un canal avantageusement dirigé sensiblement axialement par rapport à longueur de la pompe.

Toutefois comme il est évident pour l'homme de l'art, le canal peut avoir toute forme, en particulier une forme coudée, de manière à assurer par exemple une projection perpendiculairement à l'axe de la pompe.

La membrane élastique peut être réalisée en tout matériau élastique bien connu dans l'état de la technique, par exemple un caoutchouc, un élastomère, et de préférence en matériaux thermo-élastiques tels que ceux commercialisés par la Société A.E.S sous la dénomination VISKAFLEX^{®}, la Société DUPONT sous la dénomination ALCRYN^{®} ou HYTREL^{®}, la Société DSM sous la dénomination SARLINK^{®} ou la Société SHELL COMPANY sous la dénomination KRATON^{®}. Ladite membrane élastique a, au niveau de l'orifice de sortie, une épaisseur suffisante pour former une valve à sens unique vers la sortie. C'est à dire que par actionnement du piston vers l'orifice de sortie, la force exercée sur le piston permet à ladite valve de s'ouvrir permettant ainsi l'expulsion du fluide. Par contre, après expulsion du liquide, si l'on procède à un retrait du piston la valve reste hermétiquement close et il se crée, au niveau du conduit de pompe une pression réduite ou un vide.

Le conduit de pompe est muni d'un orifice d'admission de fluide, permettant au fluide de venir remplir celui-ci. Cet orifice d'admission peut avoir toute forme, arrondie, allongée, peut se présenter sous forme d'un canal, d'une tranchée, d'une rainure, etc ...

Telle une seringue, la pompe selon la présente invention comprend un piston mobile installé dans le corps de la pompe ; le piston est de préférence installé dans la direction de la longueur du dispositif. Par piston "mobile", l'on entend mobile par rapport au corps dans lequel il est logé, sans préjuger lequel, du piston ou du corps, bouge. Ce piston peut se déplacer entre une position en butée située vers l'orifice de sortie du fluide, et une position au delà de l'orifice d'admission de fluide. La butée peut être de préférence, comme dans une seringue classique, l'extrémité du conduit de pompe du côté de la sortie, cependant, on peut ménager une autre butée, si désiré, avant cette extrémité.

Dans le premier cas, après expulsion du fluide par actionnement relatif du piston et du corps de pompe, le volume de fluide retenu entre la valve de sortie et l'extrémité du piston sera réduit au seul volume du canal d'évacuation. Dans le second cas, en plus du canal d'évacuation, le fluide occupera un certain volume du conduit de pompe.

Comme dans une seringue classique, l'extrémité du piston de la pompe selon la présente invention est ajustée hermétiquement à frottement doux au cylindre de pompe.

On comprend donc que lorsque l'on tire le piston en direction opposée de l'orifice de sortie, l'on crée une pression réduite dans le conduit de pompe, ladite pression réduite étant "cassée" lorsque l'extrémité du piston arrive au niveau de l'orifice d'admission de fluide. A ce moment, le fluide est aspiré à l'intérieur du conduit de pompe qu'il remplit.

Pendant le déplacement relatif du piston vers l'orifice de sortie, lorsque le piston dépasse l'orifice d'admission, il emprisonne ainsi dans le conduit de pompe un certain volume de fluide. Le volume déterminé entre cette position et la position la plus extrême, en butée du piston mobile, correspond à la quantité de fluide prédéterminée qui sera expulsée par la pompe.

La compression du fluide par le piston, ladite compression étant réalisée par exemple et de préférence à l'aide d'un moyen élastique ou par pression à l'aide du pouce sur le piston, permet l'ouverture de la membrane élastique formant valve et l'expulsion du fluide.

On comprend que l'orifice d'admission de fluide doit avoir une taille suffisante car, si celle-ci était de faible taille, lors du déplacement du piston, une certaine quantité de fluide pourrait être expulsée par la sortie sans avoir le temps de retourner vers le reste du contenu du flacon.

C'est pourquoi, lorsque l'on veut un dosage très précis de la quantité de fluide expulsé, de préférence l'orifice d'admission est de taille suffisante pour que seule la quantité de fluide déterminée puisse se trouver emprisonnée dans l'extrémité du conduit de pompe en vue de son expulsion par l'orifice de sortie.

Comme on le voit de ce qui précède, la position de repos de la pompe selon l'invention est la position où le piston se trouve en butée. C'est pourquoi de préférence la pompe selon l'invention est pourvue d'un moyen élastique de rappel du piston en position en butée. Ces moyens élastiques sont bien connus de l'homme de l'art et sont tels qu'un ressort, ledit ressort étant installé à l'intérieur ou à l'extérieur de la pompe, dans l'axe du déplacement du piston ; ledit ressort peut être réalisé en un matériau métallique ou une matière plastique, la nature du ressort étant adaptée au fluide contenu dans le bocal lorsque ledit ressort est installé à l'intérieur du flacon en contact avec le fluide.

Ces moyens élastiques peuvent être constitués notamment par l'enveloppe du flacon élastique elle-même comme illustré ci-après par les figures. Celle-ci peut par exemple comporter une partie en accordéon, ou en convexité annulaire d'épaisseur suffisante pour former moyen de rappel. L'enveloppe est par exemple à ce niveau solidaire d'une part du corps de pompe et d'autre part du piston par l'intermédiaire d'anneaux dont peuvent être munis ces éléments. Ces anneaux peuvent coopérer avec les rainures correspondantes dont est munie dans ce cas l'enveloppe. Afin de renforcer les moyens de rappel on peut par exemple utiliser, si désiré, par exemple deux éléments de rappel tels que des accordéons situés notamment de part et d'autre d'un anneau de maintien, solidaire du piston comme on le verra illustré ci-après.

La membrane élastique peut être une pièce distincte du flacon élastique. Cependant, dans des conditions préférentielles de mise en oeuvre de la pompe ci-dessus décrite, la membrane élastique et le flacon élastique sont une pièce unique. Le nombre de pièces de la pompe selon l'invention peut donc être remarquablement réduit. On peut en effet, selon l'invention, avoir un flacon élastique muni d'une pompe comportant uniquement trois pièces : tout d'abord un flacon en matériau élastique constituant à la fois le récipient du fluide, la membrane formant valve à sens unique et les moyens élastiques de rappel de la position relative corps de pompe-piston en position de repos ; les deux autres pièces sont le corps de pompe, ainsi que le piston.

Dans d'autres conditions préférentielles, le flacon élastique selon la présente invention est muni d'au moins une partie telle qu'un épaississement, adaptée au remplissage du flacon à l'aide d'une aiguille creuse, de telle sorte qu'après perçage, remplissage et retrait de ladite aiguille creuse, le percement se trouve hermétiquement fermé.

De préférence, ladite partie spécialement conçue est un épaississement de la paroi du flacon élastique. Cet épaississement peut être ponctuel, comme illustré ci-après, annulaire ou général.

Il est évident pour l'homme de l'art qu'un épaississement ponctuel est préféré, de manière notamment à économiser la quantité de la matière première utilisée pour la fabrication de l'enveloppe ; si l'on veut privilégier la facilité de remplissage en évitant d'avoir à orienter la pompe dans ce but, un épaississement annulaire sera préféré.

Une pompe préférée selon l'invention comprend un corps de pompe muni d'un anneau frontal, installé à proximité de la sortie de fluide. Un tel anneau frontal permet la fixation du flacon élastique au corps de pompe, de manière hermétique. Un tel anneau permet aussi la réalisation de moyens élastiques de rappel du piston en position de butée.

Le corps de pompe comprend de plus de préférence un anneau postérieur, lequel peut remplir plusieurs fonctions. D'une part, dans le cas d'un dispositif à utilisation manuelle, cet anneau postérieur peut servir à la préhension par les doigts de l'utilisateur par exemple. Cet anneau postérieur peut également, dans le cas d'une utilisation d'un ressort de rappel, servir au montage de celui-ci. Enfin dans le cas où la pompe selon l'invention est un dispositif constitué uniquement de trois pièces, cet anneau postérieur peut coopérer avec l'enveloppe du flacon élastique, au niveau postérieur d'un accordéon constituant des moyens élastiques de rappel.

Le piston d'une pompe selon la présente invention est également de préférence muni d'un anneau permettant, soit dans le cas d'une utilisation manuelle, la préhension du piston par exemple par les doigts de l'utilisateur. Cet anneau peut aussi coopérer avec l'enveloppe du flacon de façon à constituer des moyens en accordéon de rappel du piston. Comme ci-dessus l'anneau peut s'engager dans les rainures dont est muni le flacon selon l'invention.

Si les éléments ci-dessus peuvent avoir toute forme adaptée, une forme circulaire, analogue à celle d'une seringue classique en verre ou en matière plastique, est tout particulièrement préférée.

L'anneau du piston ci-dessus est de préférence un anneau incomplet.

Une telle conformation permet un montage du piston dans le corps de la pompe comme on le verra expliqué plus en détail dans les figures.

Dans des conditions préférentielles, le piston ci-dessus a la conformation générale d'un élément allongé, correspondant à un piston classique, ledit élément allongé étant muni d'une pluralité, de préférence trois, éléments en forme d'ancre marine, comportant donc chacune un élément radial, à l'extrémité duquel est installé un élément en forme d'arc. La pluralité d'arcs forme alors l'anneau incomplet cité ci-dessus. Dans un tel cas, par exemple, le corps de pompe est constitué d'un élément cylindrique, comportant un à son extrémité antérieure un anneau et à son extrémité postérieure un autre anneau cylindrique, de diamètre plus important que le diamètre des arcs ci-dessus décrits, le fond antérieur de l'anneau cylindrique postérieur étant évidé de telle sorte que les ancres ci-dessus puissent traverser le fond dudit cylindre permettant ainsi, par l'intermédiaire de rainures correspondant aux éléments radiaux ci-dessus, et ménagées dans le cylindre constituant le corps de pompe, le déplacement longitudinal du piston.

Les rainures aménagées dans le corps de pompe remplissent deux fonctions : d'une part elles permettent le déplacement du piston, par glissement des éléments radiaux dans l'axe desdites rainures, et l'extrémité des rainures du côté antérieur constitue l'orifice d'admission de fluide permettant à celui ci d'atteindre le conduit final de pompe correspondant au volume prédéterminé de fluide à expulser.

La dynamique d'une pompe selon la présente invention est la suivante :

Considérons que la position d'équilibre est la position dans laquelle le piston se trouve en butée. Comme il est clair pour l'homme de l'art, les déplacements indiqués dans la présente demande en général sont des déplacements relatifs. En effet de préférence, on peut maintenir fixe le piston, et déplacer le corps de pompe comme illustré ci-après, ou encore maintenir fixe le corps de pompe et déplacer le piston pour obtenir la sortie du fluide.

Lorsque le piston recule, celui-ci détermine une cavité dont l'état de pression est un vide partiel, en effet l'orifice de sortie est bouché par la membrane élastique, empêchant l'entrée de l'air dans la pompe. En reculant encore, le piston finit par arriver au niveau d'un orifice d'admission de fluide. A ce moment le conduit de pompe se remplit rapidement de fluide. On peut alors repousser, ou laisser repartir le piston en position de butée. Lorsque le piston arrive à nouveau au niveau de l'orifice d'admission du fluide, en arrivant à l'extrémité de celui-ci, il emprisonne un volume prédéterminé du fluide. Le volume entre cette position extrême et la butée du piston, détermine alors la quantité de fluide expulsée. A partir de ce moment a lieu l'éjection du fluide.

Une pompe selon l'invention a de nombreux avantages qu'elle confère également à un flacon élastique muni d'une telle pompe.

On peut ajuster le volume prédéterminé proposé pour la pompe en jouant tant sur la surface de section de la cavité ou conduit de pompe, que sur la longueur de cette cavité en modifiant la profondeur de l'orifice de l'admission. Le dosage est constant et ne dépend ni de la pesanteur ni de la rapidité d'activation de la pompe. Il ne peut dépendre que de l'effet de ressort que l'on donne au mouvement relatif corps de pompe-piston, et ceci pour une viscosité et un diamètre des orifices donnés.

La paroi du flacon peut jouer le rôle à la fois de récipient et de ressort de rappel réduisant ainsi un flacon-pompe selon l'invention à un minimum de pièces, lequel minimum peut être de trois pièces seulement pour l'ensemble.

La pompe selon l'invention est très simple à assembler notamment dans le cas où elle ne comprend qu'un piston dans un corps, le tout installé dans une enveloppe élastique.

La pompe assure la projection des gouttes ; de ce fait elle ne nécessite pas d'orientation particulière de la surface réceptrice ou de celui qui projette les gouttes. Dans le cas par exemple de l'utilisation pour un usage ophtalmique, une telle pompe dispense le patient d'incliner la tête en arrière : il peut tout aussi bien l'incliner en avant ou rester en position droite.

Le fait que les gouttes soient projetées rend possible également l'utilisation de cette pompe en état d'apesanteur.

L'utilisation d'une paroi élastique pour l'enveloppe permet de réaliser en une pièce notamment les fonctions suivantes :
- une fonction valve à sens unique, permettant le fonctionnement sans réaspiration d'air ou de la substance délivrée elle-même ; la pompe permet ainsi de dispenser des formulations sans conservateur qui peuvent être utilisées de façon répétée sans risque de contamination de l'intérieur du flacon.
- une fonction flacon ; l'élasticité de la paroi permet en effet à la paroi de s'écraser graduellement au fur et à mesure que le liquide est évacué par la pompe. La paroi élastique autorise l'injection à travers elle-même d'un fluide, particulièrement à travers des lieux aménagés à cet effet : soit deux membranes épaissies à perforer à l'aide d'une aiguille creuse, soit d'un dispositif d'injection lui-même inséré dans le moule au moment de la fabrication de l'enveloppe élastique pour que l'injection de cette matière élastique dans le moule assure une parfaite étanchéité entre le dispositif d'injection et la paroi elle-même. L'injection et le remplissage peuvent être réalisées au moyen d'aiguilles ou de trocarts. Elle pourrait être également réalisée soit par dilatation d'un anneau extensible spécialement dessiné, soit par l'application d'une augmentation de la pression sur la paroi au niveau d'un dispositif d'injection inséré par le moulage à cet effet.

L'injection peut être suivie du simple retrait de l'aiguille, du trocart ou de tout autre moyen d'injection, du fait de l'élasticité de cette paroi et de son retour à une position d'étanchéité.

L'étanchéité peut encore être améliorée par exemple par un thermoscellage ou encore un scellage à l'aide de tout autre moyen : ultrasons, radio, radiations cohérentes, etc ..., ce qui assure une étanchéité parfaite au niveau où a été réalisée l'injection.

L'injection ou le remplissage d'une telle pompe peut être associée à une aspiration qui précède et/ou accompagne et/ou suit le remplissage de façon à éliminer tout gaz résiduel dans le flacon après remplissage.

La dose délivrée à chaque activation de la pompe ne varie pas, quelle que soit la pression de l'environnement, puisqu'aucun gaz n'existe à l'intérieur du système, lequel système est de plus dépourvu d'un espace mort.

Le dispositif selon la présente invention autorise également un remplissage étanche dans le récipient ainsi constitué, sans air, sans gaz, et quelque soit la pression ambiante, et sans que l'étanchéité ne souffre des variations successives éventuelles de la pression de l'environnement. Ce remplissage peut être réalisé stérilement sans aucun contact du fluide, liquide ou gel, avec le milieu environnant ; il peut donc être réalisé notamment à l'abri total de l'air ou de gaz du remplissage jusqu'à l'utilisation finale. Un tel remplissage peut être réalisé par injection des fluides à l'aide d'une aiguille creuse au niveau des épaississements précités. On opère de préférence alors sous atmosphère contrôlée sur le plan microbiologique, par exemple par utilisation de flux laminaire.

De plus, puisque les parties flexibles sont des parties élastiques, un tel dispositif assure un minimum de particules.

Enfin, comme on le voit, une telle pompe permet de se dispenser d'un propulseur, notamment d'un propulseur fluoré ou de tout autre gaz dont les variations de température feraient varier les doses, et dont la présence imposerait une paroi additionnelle pour éviter au propulseur de se mélanger au principe actif.

La pompe selon l'invention trouve de remarquables applications, notamment en ophtalmologie. Elle peut notamment être munie d'un boîtier tel que celui décrit dans US-A-5 267 986, dont l'enseignement est introduit ici par référence, et permet aussi la réalisation d'un dispositif permettant de projeter un fluide dans le cul de sac conjonctif de l'oeil en ne provoquant substantiellement pas de sensation douloureuse ou d'inconfort, en raison de la réserve d'énergie très précise pouvant être conférée aux gouttes propulsées.

C'est pourquoi la présente invention a aussi pour objet une pompe pour projeter un volume régulièrement reproductible d'un fluide dans le cul de sac conjonctival d'un oeil en ne provoquant substantiellement pas de sensation douloureuse ou d'inconfort, comprenant un réservoir en matériau élastique, un corps de pompe, un piston mobile dans le corps de pompe, et un moyen élastique de rappel du piston en butée, ledit moyen élastique de rappel étant calibré de manière à éviter une sensation douloureuse ou d'inconfort lors de la projection du fluide.

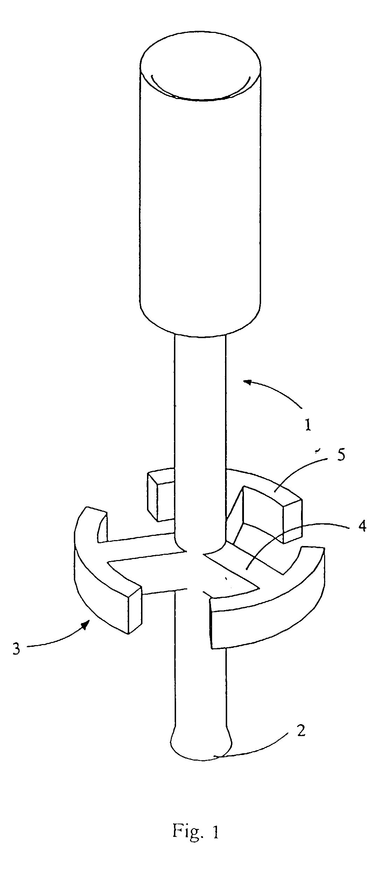

L'invention sera mieux comprise si l'on se réfère aux dessins annexés, sur lesquels la figure 1 représente, en perspective, un piston utilisé dans la configuration d'une pompe-flacon en trois pièces.

La figure 2 représente également en perspective un corps de pompe, destiné à coopérer avec le piston ci-dessus.

La figure 3 représente toujours en perspective, et en coupe, le piston installé dans son corps de pompe.

La figure 4 représente, toujours en perspective, une enveloppe destinée à coopérer avec les deux éléments ci-dessus constituant, avec les deux éléments ci-dessus, un flacon-pompe.

La figure 5 représente en coupe le montage des trois éléments ci-dessus.

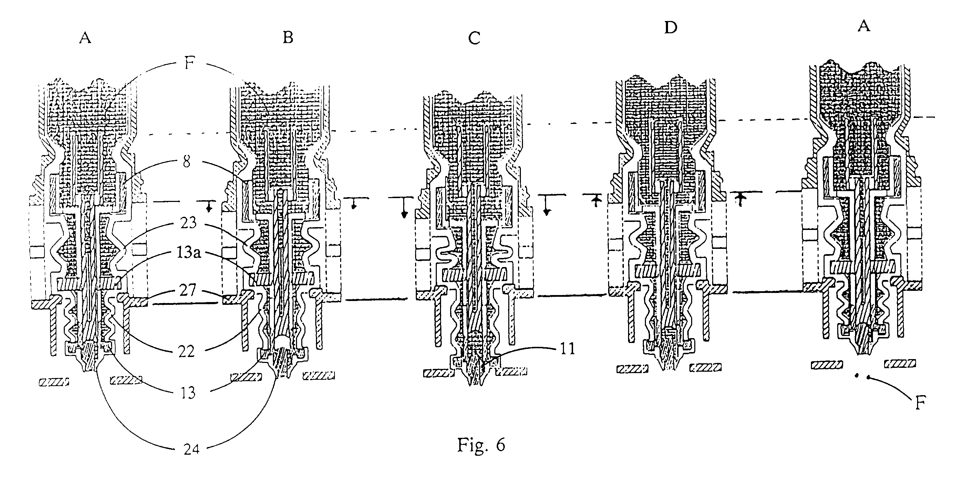

La série de figures 6 représente en coupe la dynamique d'une pompe selon l'invention.

Sur la figure 1 on observe un piston constitué
- d'un grand axe longitudinal 1 à l'extrémité antérieure duquel se situe un bourrelet 2 destiné à assurer l'étanchéité de la cavité du corps de pompe lorsque ce piston y augmente la pression.
- d'ailerons 3 en forme d'ancre, ici au nombre de trois dans cette configuration. Chacun de ceux-ci est composé d'un rayon 4 au bout duquel se trouve un arc 5 ; dans la configuration ci-après, trois rayons et, au bout de chaque rayon, un arc, donc, au total, trois arcs.

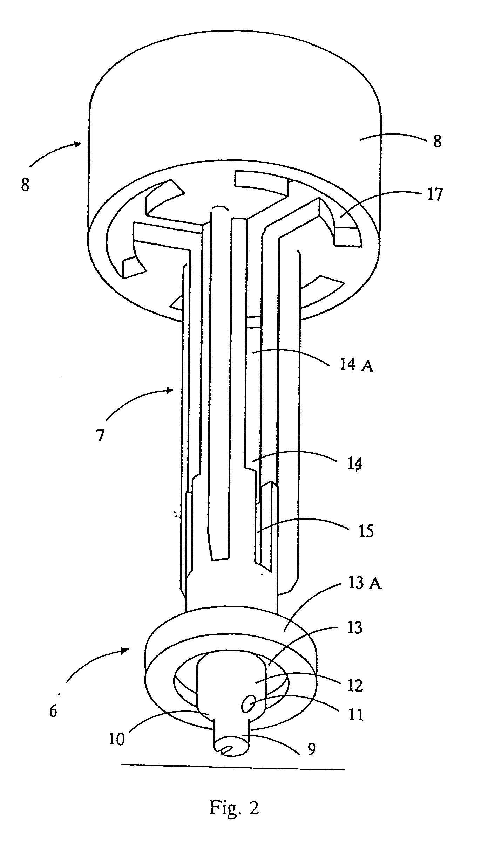

La figure 2 représente un corps de pompe, constitué de trois parties principales : la partie antérieure, ou "nez" 6 ; la partie médiane, ou "manche" 7 ; et la partie postérieure ou "corps" 8 de la pompe proprement dit.

Le nez 6 peut avoir un configuration purement cylindrique ou tronconique ; ici, il est fait d'un petit cylindre 9 tout antérieur suivi d'une zone tronconique 10, elle-même perforée de l'orifice d'évacuation 11 de la pompe. A l'arrière de la partie tronconique se trouve une autre partie cylindrique 12 et, derrière cette partie cylindrique 12, une gorge annulaire 13 servant à l'étanchéité de l'enveloppe élastique dont nous reparlerons plus tard ; cette gorge annulaire sépare le nez proprement dit d'un disque ou "disque frontal" 13a.

La manche 7 est une manche cylindrique à l'intérieur de laquelle se trouve la cavité de la pompe. La paroi cylindrique de la manche 7 est perforée de rainures 14 longitudinales dans lesquelles coulissent les rayons 4 du piston : ici, trois rainures - une pour chaque rayon du piston.

Chaque rainure a deux portions :
- une portion postérieure plus large 14 pour le coulissement des rayons du piston,
- une portion antérieure plus étroite réalisant l'orifice de communication entre le liquide extérieur et la cavité de la pompe et constituant un orifice d'admission 15. La hauteur de cette cavité de la pompe détermine le niveau à partir duquel le piston effectuera une compression sur le fluide. Elle détermine donc le volume de la dose à éjecter.

Sur la paroi intérieure de cette manche, dans sa partie toute frontale faisant suite au nez de la pompe, se trouve une butée 16 comprenant une contre-dépouille annulaire que l'on verra sur la figure suivante, dans laquelle vient se loger le bourrelet annulaire du piston dans la position de repos ou position fermée de la pompe. Cette contre-dépouille 16 permet au bourrelet annulaire 2 antérieur du piston d'exercer une très discrète compression après l'assemblage initial, de façon à maintenir le reste de la pompe en parfaite occlusion sans provoquer le fluage du bourrelet antérieur du piston pendant le stockage de la pompe avant sa période d'utilisation. Il est ainsi impossible à l'air ou au liquide contenu dans l'orifice d'évacuation 11 du nez, ici un canal d'éjection, de rentrer en contact avec le liquide contenu dans le reste de la pompe ou du flacon.

Le corps de la pompe 8 est fait d'une cavité cylindrique en continuité avec la manche, et d'un diamètre nettement plus grand, et sera logé lui-même dans l'anneau postérieur de l'enveloppe pour l'activation de la pompe.

On trouve dans la partie antérieure de cet élément les évidements 17 permettant le passage des ailerons 3 en vue d'installer le piston dans le corps de pompe.

Dans le cas illustré ici, c'est le corps de pompe qui est mobile, alors que le piston sera tenu en position fixe.

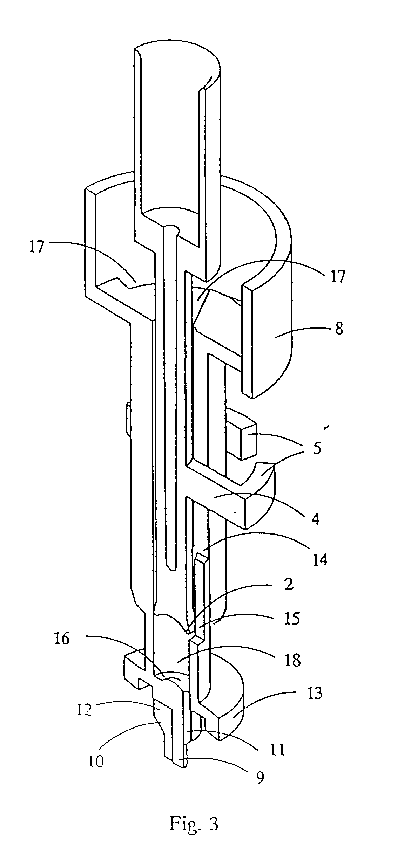

La figure 3 représente le piston installé dans le corps de pompe. En plus de certains des éléments ci-dessus, on peut observer la butée antérieure 16 du piston munie d'une contre-dépouille. On peut observer également un orifice d'admission, et la position du piston dans le corps est telle que si celui-ci se déplace vers l'avant, il va bloquer, dans la cavité 18 ou conduit de pompe, le volume prédéterminé de fluide admis par l'orifice 15. On peut aussi observer un rayon 4, installé dans une rainure longitudinale dans lequel il est capable de coulisser. Du côté postérieur du corps de pompe, on peut observer également un évidement 17 ayant permis le passage d'un aileron. On peut donc observer que, par rapport à une seringue classique, la pompe et le corps sont munies de trois parties annulaires 12, 5 et 8.

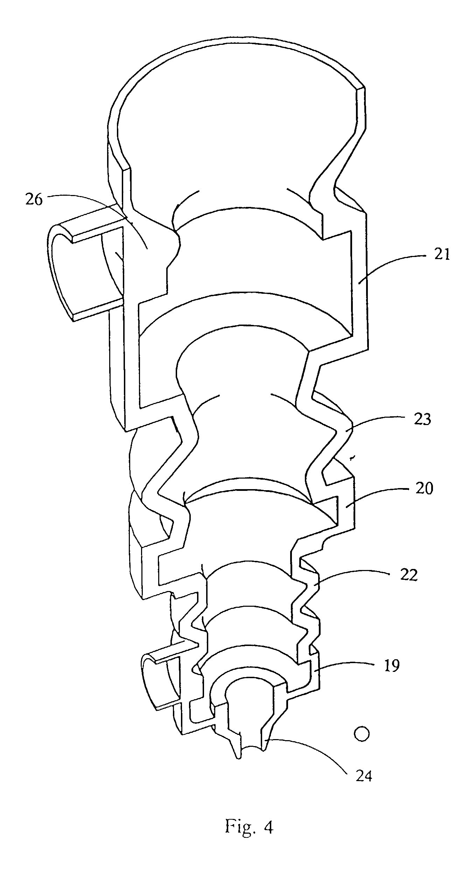

L'enveloppe élastique est représentée sur la figure 4, elle comprend trois anneaux : un anneau frontal 19, un anneau médian 20 et un anneau postérieur 21, limitant entre eux un accordéon antérieur 22 et un accordéon postérieur 23. L'anneau frontal 19 coopère avec l'anneau 13a du corps de pompe, l'anneau médian 20 coopère avec l'anneau incomplet formé par les arcs 5 dont est muni le piston, et l'anneau postérieur 21 coopère avec l'anneau postérieur 8 du corps de pompe. Les anneaux de l'enveloppe élastique bloquent fortement les anneaux des deux autres pièces ; en particulier l'assemblage au niveau des anneaux 13a et 19 est parfaitement hermétique. De plus on observe au niveau le plus antérieur de l'enveloppe, la membrane élastique 24 formant valve à sens unique vers la sortie qui comprend les parties complémentaires du petit cylindre 9, de la zone tronconique 10.

On observe également que cette enveloppe comprend deux parties qui ont été spécialement conçues pour permettre le passage d'aiguilles creuses en vue du remplissage du flacon-pompe par un fluide, liquide ou gel, il s'agit des zones 25 et 26. Celles-ci ont en effet une épaisseur plus importante que celle des zones vicinales. De plus ces zones comportent chacune un petit cylindre pouvant par exemple être thermoscellé sous pression entre deux mâchoires chauffantes. Un tel dispositif cylindrique peut être remplacé par exemple par une surépaisseur plus importante et en relief vers l'extérieur de l'enveloppe, faisant donc saillie sur la paroi extérieure, et sur laquelle on peut appliquer une pièce chauffée de manière à faire fondre ce relief pour sceller de façon sûre l'orifice ayant permis la pénétration d'une aiguille.

Enfin, il est à noter que sur la figure 4, la partie postérieure de l'enveloppe faisant uniquement office de récipient n'a pas été ici représentée.

Une telle pompe est assemblée comme suit : le piston est assemblé tout d'abord dans le corps de pompe jusqu'à ce que le bourrelet annulaire antérieur arrive en butée. La pompe est ainsi en position fermée de repos. On installe alors la pompe dans l'enveloppe élastique tandis que des jets de gaz comprimé dilatent l'enveloppe élastique pendant l'assemblage permettant celui-ci avec un minimum de friction.

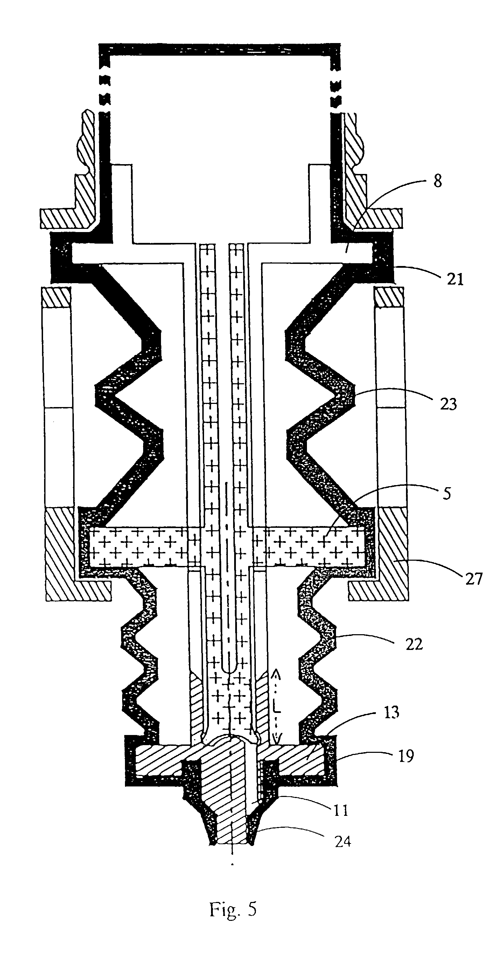

La figure 5 schématise un assemblage de trois pièces analogues aux pièces ci-dessus, mais comportant quelques différences mineures de détails. Sur cette figure, on peut essentiellement observer l'installation des anneaux 13a, 5 et 8 respectivement dans les anneaux de l'enveloppe numérotés 19, 20 et 21.

On peut également observer les ressorts accordéons antérieur 22 et postérieur 23.

Comme il est clair sur cette figure, la longueur L correspond au recul du piston dans le corps permettant d'une part l'introduction de fluide dans le conduit 18 de pompe et d'autre part déterminant en fonction du diamètre intérieur du conduit 18, le volume de fluide destiné à être expulsé.

Sur la figure 5, on peut observer également que la pompe selon l'invention a été installée dans une coque rigide 27. On distingue également plus clairement la contre-dépouille annulaire. Dans le cas illustré ci-dessus, dans le cas de la projection de doses unitaires d'un liquide ophtalmique, des dimensions peuvent être par exemple les suivantes :
- diamètre du canal constituant l'orifice de sortie, et sa longueur : respectivement 0,60 mm et 3,0 mm environ.
- épaisseur de l'enveloppe de kraton au niveau de la valve 24 : environ 0,8 mm allant en diminuant vers l'extrémité de sortie du fluide.
- épaisseur de l'enveloppe de kraton au niveau de l'accordéon 23 : 1 mm, et à l'accordéon 22 : 0,75 mm.

On peut enfin observer que la partie postérieure de l'enveloppe, en haut sur la figure, a été refermée par exemple par scellage, pour que le corps de pompe et le piston soient totalement enveloppés à l'exception de l'extrémité antérieure du corps de pompe.

Les figures 6 montrent la dynamique de la pompe. Sur cette série de figures, la pompe a été installée comme sur la figure 5 dans une coque rigide. Avec l'utilisation d'une telle coque rigide, c'est le corps de pompe qui est mobile alors que le piston est tenu en position fixe.

F représente le fluide dont a été remplie l'enveloppe élastique.

La dynamique de la pompe est la suivante :

En position 6A de repos, le piston est bloqué en position fixe par le réceptacle 27 de la pompe, c'est à dire par une structure différente des trois éléments du flacon-pompe lui-même. Il est maintenu en blocage par la compression du ressort accordéon postérieur 23.

Sur la figure 6B, lors de l'activation de la pompe, le corps de pompe est poussé en avant par sa partie postérieure 8 et il transmet cette poussée au nez qui lui est solidaire par l'intermédiaire de la manche. Cela a pour effet de créer une cavité de gouttes dans le conduit 18 de pompe dans cet espace, resté virtuel pendant la période de repos de la pompe et qui est ensuite d'un volume déterminé par la hauteur de la lèvre inférieure de la gorge antérieure 14 de la manche 7, qui met cette cavité de gouttes 18 en communication avec la cavité limitée de l'accordéon antérieur. Cette cavité de gouttes 18 est limitée en avant par la butée 16 de la pompe, sur les côtés par la partie cylindrique antérieure 18 de la manche non-ouverte par les gorges latérales 14 et, en arrière, par la partie antérieure 2 du piston elle-même limitée par le bourrelet annulaire 2 antérieur de ce piston.

Figure 6 C, lorsque le nez est poussé suffisamment en avant pour que le bourrelet antérieur 2 du piston se trouve alors en arrière des lèvres antérieures 15 des gorges antérieures de la manche 7, la dépression dans la cavité de gouttes 18 est alors comblée par l'arrivée du fluide F. La pompe est dite alors en position chargée ou ouverte.

Cette position chargée peut être gardée bloquée par un système d'encliquetage du réceptacle 27 qui lui-même sera débloqué par la pression sur une gâchette par l'utilisateur. La gâchette fait partie du boîtier réceptacle 27 dans lequel cette pompe est logée.

Durant l'activation, l'accordéon ressort postérieur 23 est sous compression et l'accordéon antérieur 22 est en extension.

Figure 6D, pendant la phase d'éjection des gouttes du fluide F, c'est à dire du retour du nez et du corps dans leur position initiale de repos, l'accordéon ressort postérieur 23 initialement comprimé se détend et l'ensemble reprend la position de repos 6E.

Le scellage et la stérilisation du flacon ci-dessus peuvent être réalisés comme suit :

La partie postérieure du moule du piston sert elle-même à l'assemblage. Cet assemblage est effectué en milieu d'air filtré contrôlé, dès la sortie du moule à chaud, et en atmosphère contrôlée. Dès le retrait du noyau du piston de la poche élastique, le scellage de la partie postérieure de l'enveloppe élastique est effectué aussitôt, soit par thermoscellage, soit par ultra-sons, radio, etc. L'intérieur de la pompe est dorénavant impossible à contaminer durant les manipulations ultérieures. Ainsi, après scellage de la partie postérieure de l'enveloppe élastique, le corps de la pompe et le piston deviennent totalement enveloppés dans cette dernière. Seule l'extrémité antérieure du corps de la pompe devient directement accessible depuis l'extérieur de l'enveloppe.

Juste avant le scellage, un gaz peut être injecté pour la stérilisation complète à l'intérieur - gaz qui sera réaspiré avant l'injection de la formulation, ceci pourra être fait sans conservateur puisque le milieu est parfaitement étanche et stérile.

Afin de minimiser le coût des installations stériles de remplissage en particulier, la stérilisation peut être effectuée après scellage par gamma radiations, électrons, par gaz, par liquide, etc ... .

La stérilisation peut aussi être effectuée avant le thermoscellage par l'injection de gaz ou d'air chaud pendant le scellage.

Si la stérilisation est effectuée par injection d'air chaud ou de liquide antiseptique, la pompe sera préalablement mise en position d'activation afin d'exposer à la décontamination le canal intra-nasal de la pompe et le bourrelet antérieur du piston.

## Revendications

1. Pompe pour la délivrance d'un fluide contenu dans un flacon élastique, comprenant :
- un corps de pompe ayant une extrémité antérieure, ou buse, du côté de la sortie du fluide, ladite extrémité antérieure comportant un orifice de sortie (11) normalement obturé par une membrane élastique (24), et se poursuivant vers l'arrière par un conduit de pompe (18) muni d'un orifice (15) d'admission de fluide,
- un piston mobile installé dans le corps de pompe, le déplacement relatif de l'extrémité (2) du piston par rapport au corps de pompe entre l'orifice (15) d'admission et une position en butée (16) située vers l'orifice de sortie (11) déterminant ainsi la quantité de fluide expulsée lors du déplacement, l'extrémité (2) du piston étant ajustée hermétiquement à frottement doux au conduit de pompe (18), l'orifice d'admission (15) étant de taille suffisante pour que seule la quantité de fluide prédéterminée puisse se trouver capturée dans l'extrémité du conduit de pompe (18) en vue de son expulsion par l'orifice de sortie (11), caractérisée en ce que le corps de pompe et le piston sont totalement enveloppés par le flacon élastique, à l'exception de l'extrémité antérieure du corps de la pompe et en ce que l'activation de la pompe est effectuée au travers du flacon élastique.

2. Pompe selon la revendication 1, caractérisée en ce que l'orifice de sortie (11) est un canal dirigé axialement par rapport à la longueur de la pompe.

3. Pompe selon la revendication 1, caractérisée en ce que la pompe est pourvue d'un moyen élastique de rappel du piston en position de butée.

4. Pompe selon la revendication 3, caractérisée en ce que un moyen élastique de rappel du piston en position de butée est constitué par le flacon élastique lui-même.

5. Pompe selon la revendication 4, caractérisée en ce que le moyen élastique de rappel du piston en position de butée est constitué par une partie en accordéon d'épaisseur suffisante pour former un moyen de rappel.

6. Pompe selon la revendication 4 ou 5, caractérisée en ce que le moyen élastique de rappel du piston en position de butée comprend deux parties en accordéon (22, 23) situés de part et d'autre d'un anneau de maintien (5) solidaire du piston.

7. Pompe selon l'une quelconque des revendications 1 à 6, caractérisée en ce que la membrane élastique (24) et le flacon élastique sont une pièce unique.

8. Pompe selon l'une quelconque des revendications 1 à 7, caractérisée en ce que le flacon élastique est muni d'au moins une partie telle qu'un épaississement, adaptée au remplissage du flacon à l'aide d'une aiguille creuse, de telle sorte qu'après perçage, remplissage et retrait de ladite aiguille creuse, le percement se trouve hermétiquement fermé.

9. Pompe selon l'une quelconque des revendications 1 à 8, caractérisée en ce que le corps de pompe est muni d'un anneau frontal (13a) et un anneau postérieur (8).

10. Pompe selon l'une quelconque des revendications 1 à 9, caractérisée en ce que le piston est muni d'un anneau (5).

11. Pompe selon l'une quelconque des revendications 6 à 10, caractérisée en ce que l'anneau (5) ou les anneaux (13, 13a, 8) sont solidaires de l'extrémité d'une partie en accordéon du flacon élastique.

12. Pompe selon l'une quelconque des revendications 1 à 11, caractérisée en ce que le piston est allongé et muni d'une pluralité d'ancres à savoir d'éléments radiaux (4) à l'extrémité desquels se trouvent des éléments en forme d'arc (5), le corps de pompe est cylindrique et comprend un anneau antérieur (13a), un anneau postérieur cylindrique (8) de diamètre plus important que celui des arcs (5) ci-dessus le fond antérieur de l'anneau cylindrique postérieur étant évidé de telle sorte que les ancres ci-dessus puissent traverser le fond dudit cylindre permettant ainsi, par l'intermédiaire de rainures correspondant aux éléments radiaux ci-dessus, et ménagées dans le cylindre constituant le corps de pompe, le déplacement longitudinal du piston.

13. Pompe selon l'une quelconque des revendications 1 à 12 pour projeter un volume régulièrement reproductible d'un fluide dans le cul de sac conjonctival d'un oeil en ne provoquant substantiellement pas de sensation douloureuse ou d'inconfort, comprenant un réservoir en matériau élastique, un corps de pompe, un piston mobile dans le corps de pompe, et un moyen élastique de rappel du piston en butée, ledit moyen élastique de rappel étant calibré de manière à éviter une sensation douloureuse ou d'inconfort lors de la projection du fluide.

## Patentansprüche

1. Pumpe zur Abgabe eines in einem elastischen Flakon enthaltenen Fluids, mit:
- einem Pumpenkörper, der ein vorderes Ende oder eine Düse auf seiner Fluidauslaßseite aufweist, wobei dieses vordere Ende eine Auslaßöffnung (11) umfaßt, die normalerweise durch eine elastische Membran (24) verschlossen ist, und der sich ferner über einen mit einer Fluideinlaßöffnung (15) versehenen Pumpenkanal (18) nach hinten fortsetzt,
- einem beweglichen Kolben, der im Pumpenkörper installiert ist, wobei die Relatiwerschiebung des Endes (2) des Kolbens relativ zum Pumpenkörper zwischen der Einlaßöffnung (15) und einer zur Auslaßöffnung (11) hin gelegenen Anschlagposition (16) die bei der Verschiebung ausgestoßene Fluidmenge bestimmt, wobei außerdem das Ende (2) des Kolbens mit geringer Reibung hermetisch im Pumpenkanal (18) sitzt und die Einlaßöffnung (15) eine Größe aufweist, die ausreicht, daß sich nur die vorbestimmte Fluidmenge im Hinblick auf ihren Ausstoß durch die Auslaßöffnung (11) im Ende des Pumpenkanals (18) aufgenommen findet, **dadurch gekennzeichnet**, daß der Pumpenkörper und der Kolben, mit Ausnahme des vorderen Endes des Pumpenkörpers, vollständig vom elastischen Flakon umgeben sind, und daß die Betätigung der Pumpe durch den elastischen Flakon hindurch erfolgt.

2. Pumpe nach Anspruch 1, dadurch gekennzeichnet, daß die Auslaßöffnung (11) ein zur Länge der Pumpe axial ausgerichteter Kanal ist.

3. Pumpe nach Anspruch 1, dadurch gekennzeichnet, daß die Pumpe mit einer elastischen Einrichtung zur Rückstellung des Kolbens in die Anschlagposition versehen ist.

4. Pumpe nach Anspruch 3, dadurch gekennzeichnet, daß eine elastische Einrichtung zur Rückstellung des Kolbens in die Anschlagposition durch den elastischen Flakon selbst gebildet wird.

5. Pumpe nach Anspruch 4, dadurch gekennzeichnet, daß die elastische Einrichtung zur Rückstellung des Kolbens in die Anschlagposition durch ein Faltenbalg-Teil mit einer ausreichenden Dicke zur Ausbildung einer Rückstelleinrichtung gebildet wird.

6. Pumpe nach Anspruch 4 oder 5, dadurch gekennzeichnet, daß die elastische Einrichtung zur Rückstellung des Kolbens in die Anschlagposition zwei Faltenbalg-Teile (22, 23) umfaßt, die zu beiden Seiten eines fest mit dem Kolben verbundenen Halteringes (5) angeordnet sind.

7. Pumpe nach einem der Ansprüche 1 bis 6, dadurch gekennzeichnet, daß die elastische Membran (24) und der elastische Flakon einstückig ausgebildet sind.

8. Pumpe nach einem der Ansprüche 1 bis 7, dadurch gekennzeichnet, daß der elastische Flakon mit mindestens einem Teil in Art einer Verdickung versehen ist, das zum Füllen des Flakons mit Hilfe einer Hohlnadel eingerichtet ist, derart, daß nach dem Durchstechen, dem Füllen und dem Zurückziehen der Hohlnadel die Durchstichstelle hermetisch verschlossen ist.

9. Pumpe nach einem der Ansprüche 1 bis 8, dadurch gekennzeichnet, daß der Pumpenkörper mit einem vorderen Ring (13a) und einem hinteren Ring (8) ausgestattet ist.

10. Pumpe nach einem der Ansprüche 1 bis 9, dadurch gekennzeichnet, daß der Kolben mit einem Ring (5) versehen ist.

11. Pumpe nach einem der Ansprüche 6 bis 10, dadurch gekennzeichnet, daß der Ring (5) oder die Ringe (13, 13a, 8) fest mit dem Ende eines Faltenbalg-Teils des elastischen Flakons verbunden ist/sind.

12. Pumpe nach einem der Ansprüche 1 bis 11, dadurch gekennzeichnet, daß der Kolben langgestreckt und mit einer Vielzahl von Ankern, d.h. radial verlaufenden Elementen (4), an deren Ende sich bogenförmige Elemente (5) befinden, versehen ist, der Pumpenkörper zylinderförmig ausgebildet ist und einen vorderen Ring (13a) sowie einen hinteren zylindrischen Ring (8) mit einem größeren Durchmesser als dem der vorgenannten Bögen (5) aufweist, wobei der vordere Boden des hinteren zylindrischen Rings so ausgespart ist, daß die vorgenannten Anker den Boden des Zylinders durchlaufen können und dabei mittels Nuten, die den vorgenannten radial verlaufenden Elementen zugeordnet und im den Pumpenkörper bildenden Zylinder ausgebildet sind, die Verschiebung des Kolbens in Längsrichtung zulassen.

13. Pumpe nach einem der Ansprüche 1 bis 12 zum Spritzen eines regelmäßig reproduzierbaren Volumens eines Fluids in den Bindehautsack eines Auges, im wesentlichen ohne ein schmerzhaftes oder unangenehmes Gefühl zu verursachen, mit einem Behälter aus elastischem Material, einem Pumpenkörper, einem in dem Pumpenkörper beweglichen Kolben, und einer elastischen Einrichtung zur Rückstellung des Kolbens bei Anschlag, wobei die elastische Rückstelleinrichtung so eingestellt ist, daß ein schmerzhaftes oder unangenehmes Gefühl während des Ausspritzens des Fluids vermieden wird.

## Claims

1. A pump for the delivery of a fluid contained in a flexible phial, characterised in that it comprises:
- a pump body having a front end, or tip, on the fluid outlet side, the said front end comprising an outlet orifice (11) normally sealed off by an elastic membrane (24), and continuing backwards through a pump duct (18) with a fluid inlet orifice (15);
- a movable piston fitted inside the pump body, the relative displacement of the end (2) of the piston in relation to the pump body between the inlet orifice (15) and a stop (16) position located towards the outlet orifice (11) thus determining the quantity of fluid expelled on displacement, the end (2) of the piston fitting hermetically by slight friction against the pump duct (18), the inlet orifice (15) being of a sufficient size for only the preset quantity of fluid to be trapped in the end of the pump duct (18) for its expulsion through the outlet orifice (11), characterized in that the pump body and the piston are totally enveloped by the flexible phial, with the exception of the front end of the pump body and in that the activation of the pump is produced through the flexible phial.

2. A pump according to claim 1, characterised in that the outlet orifice (11) is a channel positioned axially along the length of the pump.

3. A pump according to claim 1, characterised in that the pump has an elastic means of returning the piston to the stop position.

4. A pump according to claim 3, characterised in that an elastic means of returning the piston to the stop position comprises the flexible phial itself.

5. A pump according to claim 4, characterised in that the elastic means of returning the piston to the stop position comprises a concertina part of sufficient thickness to form a means of return.

6. A pump according to claim 4 or 5, characterised in that this elastic means of returning the piston to the stop position comprises two concertinas (22, 23) located either side of a retaining ring (5) integral with the piston.

7. A pump according to any one of claims 1 to 6, characterised in that the elastic membrane (24) and the flexible phial form a single piece.

8. A pump according to any one of claims 1 to 7, characterised in that the flexible phial has at least one part such as a thickening, adapted to filling the phial with the aid of a hollow needle, of such a type that after piercing, filling and withdrawing the said hollow needle, the piercing is hermetically sealed.

9. A pump according to any one of claims 1 to 8, characterised in that the pump body has a front ring (13a) and a rear ring (8).

10. A pump according to any one of claims 1 to 9, characterised in that the piston has a ring (5).

11. A pump according to any one of claims 6 to 10, characterised in that the ring (5) or rings (13, 13a, 8) are integral with the end of a concertina of the flexible phial.

12. A pump according to any one of claims 1 to 11, characterised in that the piston is an elongated element having a plurality of anchors, i.e. radial elements (4), at the end of which are arc-shaped elements (5), the pump body is cylindrical and comprises a front ring (1 3a), a cylindrical rear ring (8), of larger diameter than the diameter of the arcs (5) described above, the front base of the rear cylindrical ring being cut away so that the above anchors can pass through the base of the said cylinder thus enabling, by means of slots corresponding to the above radial elements, and made in the cylinder comprising the pump body, the longitudinal displacement of the piston.

13. A pump according to any one of claims 1 to 12 for projecting a consistently reproducible volume of fluid into the conjunctival sac of the eye without essentially causing any sensation of pain or discomfort, comprising a reservoir made of elastic material, a pump body, a piston which can move inside the pump body and an elastic means for returning the piston to the stop position, the said elastic means of return being calibrated so as to avoid a sensation of pain or discomfort on projecting the fluid.
